Europäisches Patentamt

European Patent Office

Office européen des brevets

(19)

(11) Veröffentlichungsnummer: **0 170 202**
**B1**

(12) # EUROPÄISCHE PATENTSCHRIFT

(45) Veröffentlichungstag der Patentschrift:
**01.06.88**

(51) Int. Cl.⁴: **C 07 C 1/24,** C 07 C 11/02

(21) Anmeldenummer: **85109276.7**

(22) Anmeldetag: **24.07.85**

(54) **Verfahren zur Herstellung von ungesättigten Kohlenwasserstoffen.**

(30) Priorität: **31.07.84 DE 3428120**

(43) Veröffentlichungstag der Anmeldung:
**05.02.86 Patentblatt 86/6**

(45) Bekanntmachung des Hinweises auf die Patenterteilung:
**01.06.88 Patentblatt 88/22**

(84) Benannte Vertragsstaaten:
**BE DE FR GB NL**

(56) Entgegenhaltungen:
**DE - B - 2 052 782**
**FR - A - 1 216 236**

(73) Patentinhaber: **BASF Aktiengesellschaft,**
**Carl-Bosch-Strasse 38, D-6700 Ludwigshafen (DE)**

(72) Erfinder: **Dockner, Toni, Dr., Grossgasse 6,**
**D-6701 Meckenheim (DE)**
Erfinder: **Krug, Herbert, Mussbacher Strasse 49,**
**D-6700 Ludwigshafen (DE)**

ACTORUM AG

## Beschreibung

Es ist bekannt, dass man aus Methanol bei Temperaturen zwischen 300 und 600°C durch Wasserabspaltung Kohlenwasserstoffe herstellen kann. Ein technisches Verfahren dieser Art zur Herstellung von aromatenhaltigen Kraftstoffen ist z.B. aus DE-OS 2 935 863 bekannt. Bei diesem bekannten Verfahren wird Methanol an einem Zeolith-Katalysator bei 370°C umgesetzt.

Es sind auch Verfahren bekannt, bei denen vorwiegend olefinhaltige Kohlenwasserstoffe aus Methanol erzeugt werden.

Man verwendet bei allen diesen Verfahren feste Katalysatoren wie Zeolithe, Aluminiumsilikate und anorg. Ionenaustauscher. Die Katalysatorsysteme belegen sich unter Umständen nach kurzen Standzeiten mit Russ und müssen deshalb häufig regeneriert werden. In vielen Fällen bedeutet auch die notwendige Vorbehandlung der Katalysatoren besondere Aufwendungen. Schwierigkeiten kann auch die Wärmeabführung bei der stark exothermen Reaktion über die Gasphase mit sich bringen. Die Folge davon ist eine Überhitzung an der Katalysatoroberfläche, die die Ursache für eine Russbeladung und Desaktivierung nach kurzer Zeit sein kann.

Es wurde nun gefunden, dass man diese Nachteile weitgehend ausschalten kann, wenn man das Methanol und/oder Dimethylether in hochsiedenden Mineralölfraktionen in Gegenwart von Phosphorsäure bei Temperatur von 300 bis 450°C umsetzt.

Die Arbeitsweise im flüssigen Medium hat gegenüber den bekannten Verfahren Vorteile. So wird z.B. der Stoffübergang und der Wärmeübergang verbessert, da sich eine technisch einfache und wirkungsvolle Form der Wärmeabführung durch Siedekühlung ergibt. Die Katalysatorherstellung und die Regenerierung entfallen und das gebrauchte Mineralöl kann in Kraftwerken unter Energiegewinnung verbrannt werden.

Die Wasserabspaltung wird bei Temperaturen von 300 bis 450°C, bevorzugt bei 320 bis 380°C durchgeführt. Als Mineralölfraktionen kann man Raffinierprodukte mit einem $Kp_{760} > 300°C$ wie Vakuumgasöl, Heizöl S oder Vakuumrückstand verwenden. die Konzentration an Phosphorsäure, die feinverteilt vorliegt, soll 1 bis 10 Gew.-%, bevorzugt 3 bis 7 Gew.-%, bezogen auf Mineralöl betragen.

Die Umsetzung kann man in Rührkesseln, Blasensäulen oder Reaktionskolonnen durchführen. Die Einsatzstoffe Methanol und/oder Dimethylehter kann man flüssig oder gasförmig in den Reaktor eingeben. Eine Verdünnung mit Wasserdampf und Inertgasen wie Stickstoff oder $CO_2$ ist möglich.

Die Reaktionswärme wird zweckmässigerweise durch Siedekühlung abgeführt, indem man eine Mineralölfraktion verwendet, die bei der Spalttemperatur siedet. Diese Ausführungsform bietet einen besonderen Vorteil.

Der Umsatz an Methanol kann je nach Belastung und Reaktorgeometrie bei 40 bis 100% liegen. Unumgesetztes Methanol und Dimethylether können in die Reaktion zurückgeführt werden. Anstelle von Methanol kann man auch Dimethylether einsetzen.

Als Reaktionsprodukt erhält man Ethylen, Propylen und Butylen, neben Methan etwas Äthan, Propan und Butan neben einer geringen Menge $C_5$ KW. Ein gewisser Anteil an Kohlenwasserstoffen von $C_1$-$C_5$ entsteht bei der thermischen Behandlung des Systems Mineralöl/Phosphorsäure unter den o.g. Reaktionsbedingungen, ohne dass dem System Methanol zugeführt wird.

Diese Bildung von $C_1$- bis $C_5$-Kohlenwasserstoffen verbessert die Ausbeuten des Verfahrens, da ein Teil der minderwertigen Mineralölfraktion in höherwertige flüchtige Kohlenwasserstoffe umgesetzt wird, die teilweise mit den aus Methanol gebildeten Kohlenwasserstoffen identisch sind. Die Trennung der Wertprodukte beider Provinienz kann in derselben anlage durchgeführt werden.

Man kann einen Teil des phosphorsäurehaltigen Mineralöls ggf. kontinuierlich ausschleusen und durch frisches Öl mit Phosphorsäure ergänzen. Das gebrauchte Öl kann nach Entfernung der Phosphorsäure durch Wasserwäsche im Kraftwerk unter Energiegewinnung verbrannt werden.

*Beispiel 1*

20 g Methanol werden stündlich aus einem Vorratsgefäss über eine Dosierpumpe nach Zufuhr von 2000 $Ncm^3$ Stickstoff in einen auf 120°C erhitzten Verdampfer dosiert und gasförmig in einen 2-l-Rührkolben aus Quarzglas unter Rühren eingeleitet.

Der Vakuumrückstand enthält 5 Gew.-% $H_3PO_4$ (85 Gew.-%) als Katalysator, die durch kräftiges Rühren fein verteilt sind.

Die Reaktionsgase werden über Kopf abgezogen und bei 0°C teilkondensiert. Man erhält stündlich 15,4 g Kondensat, das aus 67,5 Gew.-% Methanol und 32,5 Gew.-% Wasser besteht.

Daneben erhält man 4,95 l Abgas mit folgender Zusammensetzung:

|  | Vol.-% | Mol-% bezogen auf umgesetztes $CH_3OH$ |
|---|---|---|
| $CH_4$ | 13,7 | 10,1 |
| $C_2H_4$ | 7,8 | 11,5 |
| $C_2H_6$ | 4,8 | 7,1 |
| $C_3H_6$ | 20,0 | 44,2 |
| $C_3H_8$ | 4,1 | 9,1 |
| $C_4H_8$ | 5,9 | 17,4 |
| $C_4H_{10}$ | 2,5 | 7,4 |
| $C_5 + C_5$ | 1,0 | 3,7 |
| $(CH_3)_2O$ | 2,3 | 3,4 |

Der Umsatz beträgt 48%. Als Olefine $C_2$-$C_4$ erhält man 73,1% des umgesetzten Methanols. Die Gesamtausbeute an Kohlenwasserstoffen bezogen auf umgesetztes Methanol beträgt 100%, die Differenz zur gefundenen Gesamtsumme ist auf gebildete Kohlenwasserstoffe aus dem Mineralöl zurückzuführen.

Erhitzt man in der gleichen Apparatur 1200 g Vakuumrückstände mit 5 Gew.-% $H_3PO_4$ (85 Gew.-%) auf 350°C ohne Zusätze von Methanol, so erhält man stündlich 0,7 l Spaltgas mit folgender Zusammensetzung:

2

| | |
|---|---|
| $CH_4$ | 17,6 Vol.-% |
| $C_2H_4$ | 9,4 Vol.-% |
| $C_2H_6$ | 3,5 Vol.-% |
| $C_3H_6$ | 32,8 Vol.-% |
| $C_3H_8$ | 8,2 Vol.-% |
| $C_4H_8$ | 20,5 Vol.-% |
| $C_4H_{10}$ | 3,5 Vol.-% |
| $C_5 + C_5$ | 5,0 Vol.-% |

### Beispiel 2

Man verfährt wie in Beispiel 1 beschrieben, setzt aber 48 g eines Gemisches aus 25 Gew.-% Methanol und 75 Gew.-% Wasser ein.

Man erhält stündlich 43 g Kondensat mit 98,6 Gew.-% Wasser und 1,4 Gew.-% Methanol neben 4,8 l Abgas mit folgender Zusammensetzung:

| | Vol.-% | Mol-% bezogen auf umgesetztes $CH_3OH$ |
|---|---|---|
| $CH_4$ | 13,1 | 7,9 |
| $C_2H_4$ | 9,9 | 12,9 |
| $C_2H_6$ | 3,7 | 4,8 |
| $C_3H_6$ | 17,1 | 30,9 |
| $C_3H_8$ | 7,6 | 13,8 |
| $C_4H_8$ | 10,9 | 26,4 |
| $C_4H_{10}$ | 5,0 | 12,1 |
| $C_5 + C_5$ | 0,8 | 2,4 |
| | | 111,6 |

Man erhält 70,2% des eingesetzten Methanols als $C_2C_4$-Olefine. Die Gesamtausbeute an Kohlenwasserstoffen bezogen auf umgesetztes Methanol beträgt 100%, die Differenz zur gefundenen Gesamtsumme ist auf gebildete Kohlenwasserstoffe aus dem Mineralöl zurückzuführen.

### Beispiel 3

Man verfährt wie in Beispiel 1 beschrieben, setzt aber 1200 g Vakuumgasöl Kp 350°C mit 5 Gew.-% $H_3PO_4$ (85 Gew.-%) ein.

Man erhält stündlich 16,2 g Kondensat, das aus 69,3 Gew.-% Methanol und 30,7 Gew.-% Wasser besteht.

Daneben erhält man 4,5 l Abgas mit folgender Zusammensetzung:

| | Vol.-% | Mol-% bezogen auf umgesetztes Methanol |
|---|---|---|
| $CH_4$ | 13,3 | 9,2 |
| $C_2H_4$ | 6,8 | 10,0 |
| $C_2H_6$ | 4,2 | 6,2 |
| $C_3H_6$ | 19,2 | 42,0 |
| $C_3H_8$ | 3,9 | 8,5 |
| $C_4H_8$ | 5,8 | 17,0 |
| $C_4H_{10}$ | 2,4 | 7,1 |
| $C_5 + C_5$ | 0,8 | 3,0 |
| $(CH_3)_2O$ | 2,0 | 3,0 |

Der Umsatz beträgt 44%. Als Olefine $C_2$-$C_4$ erhält man 69% des umgesetzten Methanols.

### Beispiel 4

Man verfährt wie in Beispiel 1 beschrieben, setzt aber stündlich 28,75 g Dimethylether ein.

Man erhält stündlich 5,7 g Kondensat mit 98,6% m/m Wasser.

Daneben erhält man 13,7 l Abgas mit folgender Zusammensetzung:

| | Vol.-% | Mol-% bezogen auf umgesetzten Dimethylether |
|---|---|---|
| $CH_4$ | 2,3 | 9,0 |
| $C_2H_4$ | 5,6 | 11,0 |
| $C_2H_6$ | 3,0 | 6,0 |
| $C_3H_6$ | 14,1 | 41,6 |
| $C_3H_8$ | 2,7 | 8,0 |
| $C_4H_8$ | 4,2 | 16,4 |
| $C_4H_{10}$ | 1,8 | 7,0 |
| $C_5 + C_5$ | 0,8 | 4,2 |
| $(CH_3)_2O$ | 51,1 | |

Der Umsatz beträgt 50%. Als Olefin $C_2$-$C_4$ erhält man 69% des umgesetzten Dimethylethers.

### Patentansprüche

1. Verfahren zur Herstellung von ungesättigten Kohlenwasserstoffen durch Wasserabspaltung aus Methanol und/oder Dimethylether, dadurch gekennzeichnet, dass man das Methanol und/oder Dimethylether in hochsiedenden Mineralölfraktionen in Gegenwart von Phosphorsäure bei Temperaturen von 300 bis 450°C umsetzt.

2. Verfahren nach Anspruch 1, dadurch gekennzeichnet, dass man die Wasserabspaltung in Mineralölfraktionen mit $Kp_{760}$ > 300°C bei Temperaturen von 320 bis 380°C in Gegenwart von Phosphorsäure vornimmt, wobei die Konzentration der Phosphorsäure 1 bis 10 Gew.-%, bezogen auf die Mineralölfraktion, beträgt.

### Claims

1. A process for the production of an unsaturated hydrocarbon by elimination of water from methanol and/or dimethyl ether, wherein the methanol and/or dimethyl ether are converted in a high boiling mineral oil fraction in the presence of phosphoric acid at from 300 to 450°C.

2. A process as claimed in claim 1, wherein the elimination of water is carried out in a mineral oil fraction having $bp_{760}$ > 300°C, at from 320 to 380°C in the presence of phosphoric acid, the concentration of the phosphoric acid being from 1 to 10% by weight, based on the mineral oil fraction.

### Revendications

1. Procédé de préparation d'hydrocarbures insaturés par élimination d'eau, à partir de méthanol et/ou de diméthyléther, caractérisé en ce que l'on fait réagir le méthanol et/ou le diméthyléther dans des fractions d'huiles minérales à haut point d'ébullition, en présence d'acide phosphorique, à des températures de 300 à 450°C.

2. Procédé suivant la revendication 1, caractérisé en ce que l'on effectue l'élimination d'eau dans des fractions d'huiles minérales de point d'ébullition, sous 760 mm de mercure, supérieur à 300°C, à des températures de 320 à 380°C, en présence d'acide phosphorique, la concentration en acide phosphorique étant de 1 à 10% en poids par rapport à la fraction d'huile minérale.